# FASCICULE DE BREVET EUROPEEN

(11) **EP 3 154 669 B1**
(45) Date de publication et mention de la délivrance du brevet: **31.03.2021**
(21) Numéro de dépôt: 15732440.1
(22) Date de dépôt: 11.06.2015
(51) Int. Cl.: B01F 17/00, C07D 307/12, C10M 129/16, A61K 31/341

(54) **COMPOSITIONS D'ÉTHERS MONO-ALKYLIQUES DE MONOANHYDRO-HEXITOLS, PROCÉDÉS DE PRÉPARATION ET LEUR UTILISATION**
ZUSAMMENSETZUNGEN VON MONOALKYL ETHERN VON MONOANHYDROHEXITOLEN, HERSTELLUNGSVERFAHREN DAFÜR UND VERWENDUNG DAVON
COMPOSITIONS OF MONO-ALKYL ETHERS OF MONOANHYDRO-HEXITOLS, PRODUCTION METHODS THEREOF AND USE OF SAME

(30) Priorité: 13.06.2014 FR 1401346
(43) Date de publication de la demande: 19.04.2017
(73) Titulaire: Tereos Starch & Sweeteners Belgium, 9300 Aalst (BE); Université Claude Bernard Lyon 1, 69622 Villeurbanne Cedex (FR); Centre National de la Recherche Scientifique (C.N.R.S.), 75016 Paris (FR)
(72) Inventeur: GOZLAN, Charlotte, 93190 Livry-Gargan (FR); DUGUET, Nicolas, F-69622 Villeurbanne Cedex (FR); LEMAIRE, Marc, F-69622 Villeurbanne Cedex (FR); REDL, Andreas, B-9300 Aalst (BE)
(74) Mandataire: Icosa
(86) Numéro de dépôt international: PCT/IB2015/054418
(87) Numéro de publication internationale: WO 2015/189796

(56) Documents cités:
- WO-A1-2012/148530

## Description

La présente invention concerne des compositions d'éthers mono-alkyliques à base de sucres, et un procédé pour obtenir de tels éthers.

Dans la littérature scientifique et technique, les molécules tensio-actives à base de sucre sont bien connues. Parmi elles, les esters d'acides gras de saccharose, les esters de sorbitane et les alkyles polyglucosides à longues chaînes ont été largement utilisés dans l'alimentation, les soins personnels, applications cosmétiques ou pharmaceutiques. Certains de ces tensioactifs ont également été largement employés au titre de nettoyants domestiques ou industriels ou comme lubrifiants.

Malgré leur utilisation et leur acceptation répandue, il est bien connu que les tensio-actifs à base d'esters ne sont stables que sur une gamme limitée de pH, tandis que les glucosides d'alkyle sont stables dans des conditions alcalines et neutres, mais pas dans des conditions fortement acides.

D'autres inconvénients sont liés aux procédés utilisés pour l'obtention de ces dérivés. En effet, dans le cas des glucosides d'alkyle de longueur de chaîne supérieure, une trans-glycosylation est nécessaire. L'utilisation d'installations assez compliquées et coûteuses est nécessaire afin d'obtenir un produit suffisamment pur. Dans le cas des esters à base de sucre, notamment les esters de sorbitane, on a besoin de solvants coûteux et toxiques, ou des températures de réaction élevées sont alors nécessaires pour obtenir les produits avec un rendement suffisamment élevé.

Afin d'améliorer la stabilité en conditions acide des composés tensioactifs à base de sucre, un éther d'alcool de sucre a été proposé récemment dans le document WO 2012/148530. Cette demande décrit un procédé pour préparer des éthers de polyols au cours duquel une masse de polyols en fusion est mise à réagir avec un aldéhyde à longue chaîne alkyle dans des conditions réductrices d'alkylation et de catalyse acide. Selon cette divulgation, des conditions réactionnelles difficiles et extrêmes sont nécessaires, ceci en combinaison avec un équipement à haute pression afin de réaliser la réaction d'alkylation réductrice. Afin d'obtenir les produits désirés, un excès d'alcool de sucre est jugé nécessaire par rapport à l'aldéhyde. Ceci induit une consommation d'énergie par mole d'éther d'alcool de sucre élevée. En outre, au terme de chaque synthèse les auteurs ont identifié par RMN¹³C le composé unique synthétisé (un seul régioisomère avec une chaine alkyle en position 6) par exemple le 2-(2-heptyloxy-1-hydroxyethyl)tetrahydrofuran-3,4-diol (exemple 1), le 2-(2-hexyloxy-1-hydroxyethyl)tetrahydrofuran-3,4-diol (exemple 2) et le 2-(2-octyloxy-1-hydroxyethyl)tetrahydrofuran-3,4-diol (exemple 3).

Par ailleurs, l'art antérieur décrit des méthodes d'obtention de monoanhydro-sorbitol. Ainsi, une méthode dans laquelle le sorbitol est dissout dans l'eau en présence d'un catalyseur acide et chauffé dans des conditions atmosphériques pendant un temps suffisant pour obtenir la teneur maximale de 1,4-sorbitane est décrite dans Acta Chemical Scandinavica B (1981) p.441-449*.* Aussi des procédés similaires ont été divulgués dans lesquels la réaction est effectuée sous pression réduite (US 2,390,395 et US 2007/173651) ou sous pression d'hydrogène modérée (US2007/173654). Dans la demande de brevet US2007/173654, un co-catalyseur de métal noble est utilisé. Néanmoins, les concentrations d'isosorbide mesurés sont assez élevées, comparativement au 1,4-sorbitane. Ainsi, les méthodes de l'art antérieur ne permettent pas d'observer un haut rendement de production de monoanhydro-sorbitol dans des conditions réactionnelles douces.

Ainsi, il est clair qu'il y a un besoin de proposer des éthers d'alcool de sucres, ayant des propriétés tensio-actives, qui peuvent être obtenus par des procédés à haut rendement qui sont à la fois, acceptables pour l'environnement, avantageux dans la consommation d'énergie et faciles à mettre en œuvre industriellement.

Ce besoin a été résolu par l'établissement d'une composition d'isomères d'éthers monoalkyliques de monoanhydro-hexitol présentant un radical éther alkyle (OR) en position C-3, C-5 ou C-6 du monoanhydro-hexitol, dans lequel le groupe alkyle (R) est un groupe hydrocarboné linéaire ou ramifié comprenant entre 4 à 18 atomes de carbone préférentiellement entre 8 et 12 atomes de carbone.

Par « radical éther alkyle (OR) en position C-3, C-5 ou C-6 » on entend un radical alkoxy substituant un groupement hydroxy (OH) porté par un atome de carbone situé en position 3, 5 ou 6 du monoanhydro-hexitol.

Par « isomères d'éthers monoalkyliques de monoanhydro-hexitol présentant un radical éther alkyle (OR) en position C-3, C-5 ou C-6 du monoanhydro-hexitol » ou « isomères en position C-3, C-5 ou C-6 d'éthers monoalkyliques de monoanhydro-hexitol », on entend les 3-alkyle monoanhydro-hexitol, 5-alkyle monoanhydro-hexitol et 6-alkyle monoanhydro-hexitol.

A titre d'exemples de groupe alkyle, on pourra citer les groupes butyl, pentyl, hexyl, heptyl, octyl, nonyl, decyl, undecyl ou dodecyl. Typiquement, le groupe alkyle est choisi parmi les groupes octyl, decyl ou dodecyl.

Plus particulièrement, la composition selon l'invention comprend au moins 1, 2, 5, 10 ou 15% (w/w) de l'un quelconque des isomères d'éthers monoalkyliques de monoanhydro-hexitol. Avantageusement, l'isomère majoritaire est le 6-alkyle monoanhydro-hexitol. Typiquement, l'isomère 6-alkyle monoanhydro-hexitol représente 34 à 98% (w/w) des isomères d'éthers monoalkyliques de monoanhydro-hexitol de la composition selon l'invention préférentiellement, 40 à 80%(w/w), plus préférentiellement, 45 à 70%(w/w). Les 3-alkyle monoanhydro-hexitol et 5-alkyle monoanhydro-hexitol peuvent être en proportions identiques ou différentes et indépendamment l'un de l'autre représenter entre 1 à 33% (w/w), préférentiellement, 5 à 30%, plus préférentiellement 10 à 27% (w/w) des isomères d'éthers monoalkyliques de monoanhydro-hexitol de la composition.

Préférentiellement, le rapport [(3-alkyle monoanhydro-hexitol + 5-alkyle monoanhydro-hexitol) / 6-alkyle monoanhydro-hexitol] est compris entre 0,02 et 2, préférentiellement, entre 0,25 et 1,8 plus préférentiellement, entre 0,4 et 1,7, entre 0,7 et 1,5 ou entre 0,8 et 1,2.

Préférentiellement, la composition selon l'invention comprend au moins 90% (w/w), de préférence au moins 95% (w/w) d'isomères d'éthers monoalkyliques de monoanhydro-hexitol.

Avantageusement, le mono-anhydro hexitol est choisi parmi le mono-anhydro sorbitol, le mono-anhydro mannitol, le mono-anhydro iditol et le mono-anhydro galactitol. Typiquement, le mono-anhydro hexitol est le mono-anhydro sorbitol ou le mono-anhydro mannitol.

Typiquement, les isomères d'éthers monoalkyliques de monoanhydro-sorbitol peuvent être de formule I dans laquelle R1, R2 et R3 sont un groupe alkyle et deux atomes d'hydrogène.

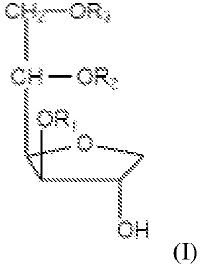

Par exemple, un isomère en C-3 d'un éther alkylique de monoanhydro- sorbitol (ou le 3- alkyle monoanhydro- sorbitol) est de formule II dans laquelle R1 est un groupe alkyle.

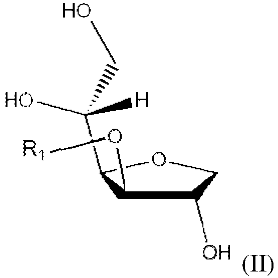

Préférentiellement, l'isomère en C-5 d'un éther alkylique de monoanhydro-sorbitol (ou le 5- alkyle monoanhydro- sorbitol) est de formule III dans laquelle R2 est un groupe alkyle.

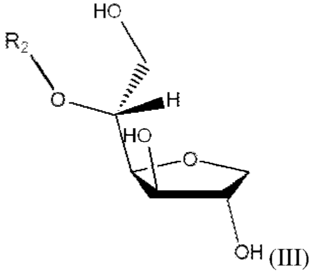

Préférentiellement, l'isomère en C-6 d'un éther alkylique de monoanhydro-sorbitol (ou le 6- alkyle monoanhydro- sorbitol) est de formule IV dans laquelle R3 est un groupe alkyle.

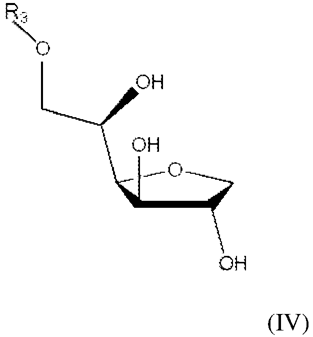

La présente invention concerne également un procédé d'obtention d'une composition d'isomères d'éthers monoalkyliques de monoanhydro-hexitol présentant un radical éther alkyle (OR) en position C-3, C-5 ou C-6 du monoanhydro-hexitol, dans lequel le groupe alkyle (R) comprend 4 à 18 atomes de carbone selon l'invention, ledit procédé comprenant les étapes suivantes :
- la déshydratation d'un hexitol pour obtenir un substrat monoanhydro-hexitol;
- l'obtention d'un acétal alkyle d'hexitan par acétalisation ou transacétalisation du substrat monoanhydro-hexitol obtenu, avec
   ∘ un réactif d'aldéhyde aliphatique comprenant de 4 à 18 atomes de carbone, par acétalisation, préférentiellement, dans un rapport de substrat/réactif entre 5:1 et 1:1, ou
   ∘ un dérivé d'un réactif d'aldéhyde aliphatique comprenant de 4 à 18 atomes de carbone, par trans-acétalisation, préférentiellement, dans un rapport substrat/réactif entre 1:1 et 1:3,
- l'hydrogénolyse catalytique de l'acétal alkyle d'hexitan, et
- la récupération d'une composition d'isomères d'éthers monoalkyliques monoanhydro-hexitol présentant un radical éther alkyle (OR) en position C-3, C-5 ou C-6 du monoanhydro-hexitol, dans lequel le groupe alkyle (R) comprend 4 à 18 atomes de carbone.

Typiquement, le procédé selon l'invention comprend en outre, au moins une étape de neutralisation, et/ou de filtration et/ou de purification après l'une quelconque des étapes a), b) et/ou d).

Préférentiellement, l'étape a) de déshydratation s'effectue par traitement de l'hexitol, par exemple sous la forme d'une masse fondue d'hexitol, avec un catalyseur acide.

Typiquement, l'étape a) s'effectue sous atmosphère d'hydrogène préférentiellement à une pression de 20 à 50 bar.

Avantageusement, l'étape a) s'effectue à une température comprise entre 120 et 170°C, préférentiellement entre 130 et 140°C.

L'étape b) d'acétalisation ou de trans-acétalisation peut être précédée d'une étape de purification du monoanhydro hexitol. La purification peut être par exemple une étape de chromatographie ou de cristallisation.

Préférentiellement, l'étape b) d'acétalisation ou de trans-acétalisation comprend :
bi) optionnellement, une première étape de préchauffage du substrat monoanhydro-hexitol, préférentiellement, à une température comprise entre 70 et 130°C, typiquement, entre 90 et 110°C.
bii) une étape d'adjonction du réactif d'aldéhyde aliphatique ou du dérivé d'aldéhyde aliphatique et
biii) une étape d'addition d'un catalyseur préférentiellement, un catalyseur acide.

Typiquement, la réaction d'acétalisation ou de trans-acétalisation s'effectue à des températures comprises entre 70 et 130°C, typiquement entre 75 et 110°C, typiquement, 77 et 110°C. Les mélanges réactionnels sont chauffés à des températures variant en fonction des réactifs et des solvants utilisés. Typiquement, pour un réactif d'aldéhyde aliphatique ou un dérivé d'aldéhyde aliphatique en C5 ou en C12, lorsque le solvant est l'éthanol la température d'acétalisation ou de trans-acétalisation peut être de 80°C, lorsque l'acétalisation ou la trans-acétalisation s'effectue en l'absence de solvant, la température réactionnelle peut être de 95°C. Le temps de réaction est déterminé par le degré de conversion atteint.

Les catalyseurs acides utilisés lors des étapes a) et b) peuvent être choisis indépendamment parmi les acides solides ou liquides, organiques ou inorganiques, des acides solides étant préférés. En particulier, les acides préférés sont choisis parmi l'acide para-toluène sulfonique, l'acide méthane sulfonique et l'acide camphosulfonique (CSA ou acide sulfonique de camphre) et les résines sulfoniques.

Lors de l'exécution de la réaction d'acétalisation ou de trans-acétalisation avec un réactif d'aldéhyde aliphatique ou un dérivé d'aldéhyde aliphatique, la réaction peut être effectuée avec ou sans solvant. Lorsque la réaction est effectuée en présence d'un solvant, le solvant est préférentiellement, un solvant polaire, typiquement un solvant polaire non-aqueux.

Lors de l'utilisation d'un solvant, celui-ci peut être choisi parmi les solvants polaires aprotiques tels que la diméthylformamide (DMF), le diméthylsulfoxide (DMSO), le diméthylacétamide (DMA), l'acétonitrile (CH₃CN), le tétrahydrofurane (THF), le 2-méthyltétrahydrofurane (2Me-THF), le cyclopentylméthyléther (CPME), le dibutyléther (DBE), le méthyl tert-butyléther (MTBE) ou encore le triméthoxypropane (TMP) ou les solvants polaires protiques tels que le méthanol (MeOH), l'éthanol (EtOH), le butanol (BuOH) ou l'isopropanol. Les solvants polaires protiques tels que l'éthanol sont particulièrement avantageux.

L'étape b) d'acétalisation peut être réalisée avec un réactif d'aldéhyde aliphatique, dans lequel le réactif aldéhyde contient de 4 à 18 atomes de carbone. Ces aldéhydes peuvent être choisis parmi les aldéhydes aliphatiques linéaires ou ramifiés. Dans un mode de réalisation préféré, les aldéhydes aliphatiques contiennent de 4 à 18 atomes de carbone, préférentiellement, 5 à 12 atomes de carbone. Certains représentants typiques des aldéhydes sont: le pentanal, l'hexanal, l'heptanal, l'octanal, le nonanal, le decanal, l'undécanal et le dodécanal.

Des travaux expérimentaux étendus ont permis de sélectionner des conditions assurant des taux de conversion et des rendements optimaux de l'étape b) d'acétalisation. Les meilleurs résultats ont été obtenus lorsque le rapport molaire du substrat au réactif est entre 5:1 et 1:1, de préférence compris entre 4:1 et 1:1, et de manière plus préférée entre 3:1 et 2:1.

L'étape b) de trans-acétalisation peut être réalisée en présence ou en absence d'un solvant afin d'obtenir des acétals cycliques alkylés à chaînes longues, à base de sucre.

Typiquement, lorsque l'étape b) de trans-acétalisation est réalisée en présence d'un solvant, le solvant préféré est l'alcool correspondant au réactif acétal utilisé.

Au cours de l'étape b) de trans-acétalisation, les dérivés d'un réactif d'aldéhyde aliphatique peuvent être les acétals de di-alkyle des aldéhydes correspondants. Les acétals de di-méthyle et les acétals de di-éthyle sont préférés.

Des travaux expérimentaux étendus ont permis de sélectionner des conditions assurant qu'au cours des réactions de trans-acétalisation, des rendements et des taux de conversion optimaux ont été obtenus lorsque le rapport molaire du substrat au réactif est entre 1 :1 et 1 :3, et de préférence entre 2 :3 et 2 :5. Les catalyseurs utilisés sont les mêmes que lors des réactions d'acétalisation.

Typiquement, l'étape c) d'hydrogénolyse de l'acétal alkyle d'hexitan peut être précédée d'une étape de filtration et/ou purification.

La purification peut être par exemple une étape de chromatographie ou de cristallisation. Préférentiellement, la purification par chromatographie est réalisée en utilisant un solvant polaire non-aqueux. Par exemple, le solvant polaire non aqueux est identique à celui utilisé dans l'étape c) d'hydrogénolyse.

Avantageusement, l'étape c) d'hydrogénolyse est réalisée à une température comprise entre 80°C et 140°C, préférentiellement à une pression entre 15 et 40 bar.

L'étape c) d'hydrogénolyse peut être réalisée avec ou sans solvant. Lorsqu'elle est effectuée en présence de solvants, les solvants peuvent être apolaires tels que par exemple l'heptane ou le dodecane. Néanmoins, les solvants polaires et plus particulièrement les solvants aprotiques et non-aqueux sont préférés puisque pour une sélectivité équivalente ils permettent une meilleure conversion que les solvants apolaires. Des exemples de solvants aprotiques sont entre autres, sans être limitant, le triméthoxypropane (TMP), le méthyltertbutyléther (MTBE), le THF, le 2Me-THF, l'éther dibutylique (DBE) et le cyclopentylméthyléther (CPME). Préférentiellement, le solvant aprotique est le CPME.

L'étape c) d'hydrogénolyse est préférentiellement réalisée dans un solvant polaire aprotique, à une température se situant entre 80°C et 140°C et une pression entre 15 et 40 bar, en présence d'un catalyseur adapté pour faire des réactions d'hydrogénolyse.

De préférence, l'étape c) d'hydrogénolyse est réalisée dans un solvant polaire non-aqueux, à une température se situant entre 100°C et 130°C et/ou à une pression entre 25 et 35 bar.

Typiquement, l'étape c) est effectuée en présence d'un catalyseur adapté tel qu'un catalyseur à base de métaux précieux, ou à base de métaux appartenant au groupe des métaux ferreux.

A titre indicatif, un catalyseur à base de métaux appartenant au groupe des métaux ferreux peut être le nickel, le cobalt ou le fer.

De préférence, l'hydrogénolyse est réalisée en utilisant un catalyseur à base de métaux précieux, comme le palladium, le rhodium, le ruthénium, le platine ou l'iridium.

Typiquement, le catalyseur utilisé dans l'étape c) peut être fixé sur un support tel que le charbon, l'alumine ou la silice. Un tel support est par exemple sous la forme de billes. Un catalyseur à base de palladium fixé sur des billes de charbon (Pd/C) est préféré.

Selon l'invention, l'hexitol tel que celui utilisé dans l'étape a) est un monosaccharide hydrogéné préférentiellement choisi parmi le sorbitol, le mannitol, l'iditol, le galactitol et leur mélange. Le sorbitol et/ou le mannitol sont préférés.

Lorsque l'hexitol est le sorbitol, le monoanhydrohexitol obtenu est le 1,4 sorbitane de formule (V).

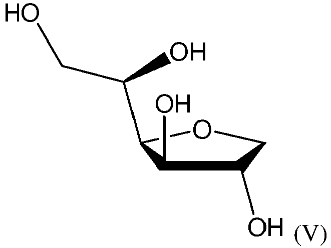

Les inventeurs ont mis en évidence que le produit intermédiaire 1,4-sorbitane pouvait être obtenu avec un bon rendement par traitement d'une masse fondue de sorbitol avec un catalyseur acide solide dans une atmosphère d'hydrogène à une pression de 20 à 50 bar, ceci à une température de réaction qui peut varier entre 120 et 170°C, pendant une période de temps suffisante afin d'obtenir un rendement optimal de sorbitane. Les températures de réaction préférées sont comprises entre 130 et 140°C.

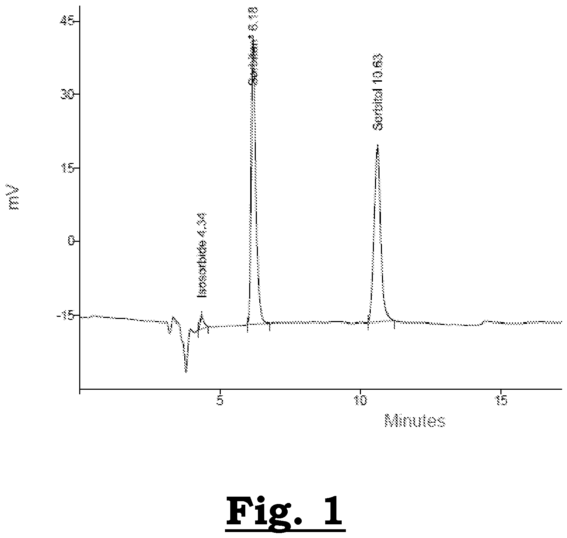

Le mélange réactionnel ainsi obtenu est constitué de 1,4-sorbitane, de sorbitol qui n'a pas réagi, de l'isosorbide et de quantités mineures de sous-produits, comme illustré sur le chromatogramme représenté sur la figure 1. Un des avantages observés ainsi est la baisse du niveau de coloration, ceci en contraste avec les procédés antérieurs classiques.

L'étape a) de déshydratation peut optionnellement être suivie d'une étape de purification du 1,4 sorbitane. Ainsi, le 1,4-sorbitane est purifié du mélange réactionnel et le reste est recyclé vers l'étape de déshydratation. Dans un mode de réalisation particulier, le 1,4-sorbitane est récupéré et purifié par cristallisation. Dans un autre mode de réalisation préféré le 1,4-sorbitane est récupéré et purifié par chromatographie. Ce 1,4-sorbitane purifié est utilisé de préférence en tant que substrat pour la réaction d'acétalisation.

Lorsque l'étape b) d'acétalisation est effectuée sans solvant, le 1,4-sorbitane est d'abord chauffé entre 90 et 110°C, puis le réactif aldéhyde est ajouté lentement, suivi de l'addition du catalyseur.

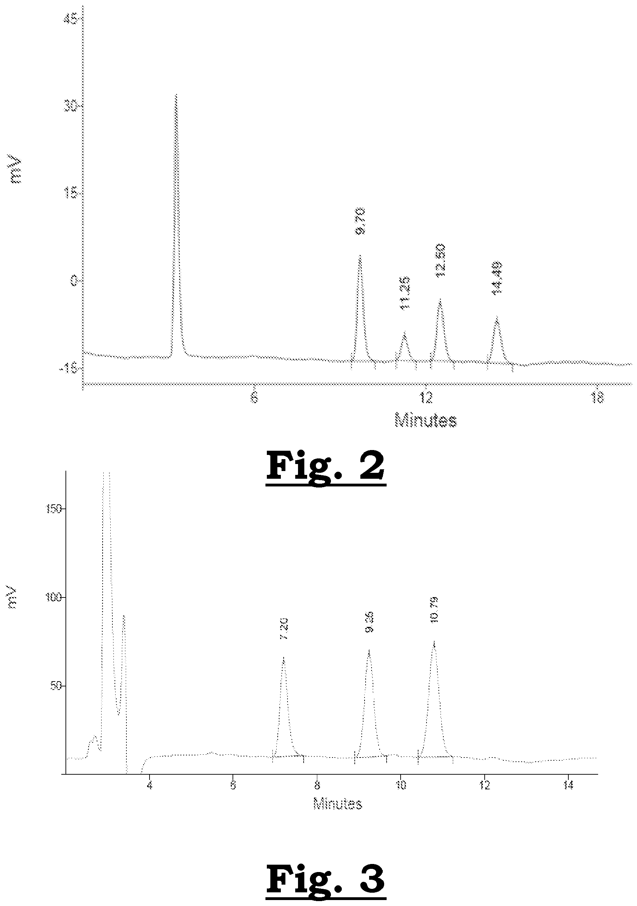

Les compositions d'acétal de sorbitane obtenus par les procédés décrits ci-dessus sont composées de 4 isomères. Ceci est illustré dans la figure 2. Deux de ces isomères correspondent à un mélange diastéréomériques d'acétal de sorbitane à 5 chaînons en position 5,6 et les deux autres isomères correspondent à un mélange diastéréomériques d'un acétal de sorbitane à 6 chaînons en position 3,5.

Les acétals de sorbitane en position 5,6 sont de formule VI dans laquelle le groupe R' est un groupe alkyle. Typiquement, R' est une chaîne aliphatique linéaire ou branchée de C3 à C17.

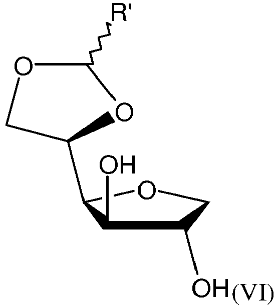

Les acétals de sorbitane en position 3,5 sont de formule VII dans laquelle le groupe R est un groupe alkyle. Typiquement, R' est une chaîne aliphatique linéaire ou branchée de C3 à C17.

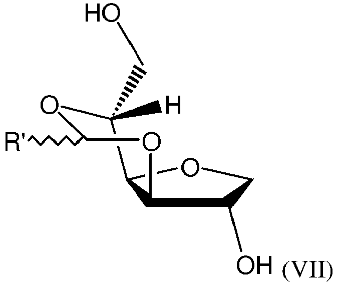

Les acétals alkyle d'hexitan obtenus ci-dessus sont ensuite soumis à une réaction d'hydrogénolyse. Ce mélange d'acétals peut être utilisé après récupération du mélange brut, ou bien après une purification par voie chromatographique. Cette réaction d'hydrogénolyse est réalisée dans un solvant polaire aprotique, à une température se situant entre 80°C et 140°C et une pression entre 15 et 40 bar, en présence d'un catalyseur adapté pour faire des réactions d'hydrogénolyse.

De préférence, l'hydrogénolyse est réalisée dans un solvant polaire non-aqueux, à une température se situant entre 100°C et 130°C et une pression entre 25 et 35 bar.

Le solvant polaire non-aqueux CPME (cyclopentyl methyl ether) a été prouvé comme étant particulièrement avantageux dans la réaction d'hydrogénolyse des acétals cycliques 5,6 et 3,5 de sorbitan.

L'invention concerne également le produit obtenu par la mise en œuvre du procédé.

L'invention concerne de plus l'utilisation de la composition selon l'invention comme agent tensioactif non ionique, émulsifiant, lubrifiant, agent antimicrobien ou agent de dispersion. Typiquement, la composition selon l'invention peut être utilisée dans un produit alimentaire ou non alimentaire ou dans un produit pharmaceutique ou cosmétique.

Lorsque la composition selon l'invention est utilisée comme un agent tensioactif non ionique, de dispersion ou émulsifiant, le produit alimentaire peut être choisi parmi les produits aérés tels que les mousses, la crème glacée, ou les produits non aérés tels que les matières grasses à tartiner ou les vinaigrettes. Le produit alimentaire peut être sous la forme d'un produit liquide choisi dans le groupe constitué par les sauces, les soupes et les boissons.

Préférentiellement, les groupes alkyles en C10-C12 sont préférés pour leur utilisation comme agent antimicrobien ou agent tensioactif non ionique.

Préférentiellement, les groupes alkyles en C5-C8 sont préférés dans l'utilisation comme agent émulsifiant, lubrifiant ou de dispersion

Sans limiter la portée de l'invention, l'invention va maintenant être davantage illustrée à l'aide d'un certain nombre d'exemples décrivant les méthodes de préparation de ces dérivés.

### FIGURES

La figure 1 : représente un chromatogramme du mélange de réaction obtenu au cours de la réaction de déshydratation selon l'exemple 1;
La figure 2 : représente un chromatogramme du mélange de réaction obtenu par trans-acétalisation sans solvant selon l'exemple 8.
La figure 3 : représente un chromatogramme du mélange de réaction obtenu par hydrogénolyse selon l'exemple 10.

### EXEMPLES

### Exemple 1 :

### La déshydratation du sorbitol:

Le D-sorbitol (20 g, 110 mmol) et 0,1% en moles d'acide camphosulfonique sont ajoutés dans un autoclave de 150 mL en acier inoxydable. Le réacteur est hermétiquement fermé, purgé trois fois avec de l'hydrogène puis l'hydrogène a été introduit jusqu'à une pression de 50 bars. Le système est ensuite chauffé à 140 °C et agité avec un agitateur mécanique pendant 15 heures. Après refroidissement à température ambiante, la pression d'hydrogène a été libérée et la mousse blanche a été diluée dans de l'éthanol (200 mL) pour obtenir un mélange homogène jaune. Le solvant est évaporé sous pression réduite et le résidu est ensuite cristallisé à partir de méthanol froid et filtré sous vide. Le matériau cristallin a été lavé avec du méthanol froid pour donner le 1,4-sorbitane (5,88 g, 35% sur théorique) sous forme d'un solide blanc. La pureté est de > 98%, telle que déterminée par HPLC, tandis que les cristaux ont montré un point de fusion de 113-114 °C. Le degré de conversion de la réaction a été déterminée à 73%, grâce à quoi on obtient un mélange de sorbitol, de 1,4-sorbitane, d'isosorbide et de quelques sous-produits en quantité très limitée, de sorte que le rapport du 1,4-sorbitane: isosorbide a été déterminé comme étant de 80: 20.

### Exemple 2:

### Acetalisation de sorbitane dans le DMF:

Dans un tube scellé, le 1,4 sorbitane (X) (0,5 g, 3 mmol) a été dissout dans du DMF (1,4 mL). Le valeraldehyde (Y) (107 µL, 1 mmol) a été ajouté goutte à goutte sous argon suivi par l'addition d'acide camphosulfonique (10 mg, 10% p / p) avant de fermer le tube. Le mélange est chauffé à 95 °C sous agitation magnétique. Après 15 heures, le mélange réactionnel foncé a été refroidi et le solvant évaporé sous pression réduite. Un degré de conversion de 95% a été atteint. Le résidu a été dilué dans de l'acétate d'éthyle et l'excédent de 1,4 sorbitane a été filtré et lavé avec de l'acétate d'éthyle. Le filtrat a été concentré sous pression réduite. Le résidu est purifié par chromatographie flash (EtOAc : cyclohexane 80:20 à 100:0) pour donner l'acétal de sorbitane (0,22 g, 89% de rendement isolé) sous forme d'une huile incolore. L'HPLC a révélé un mélange de 4 isomères.

### Exemple 3:

Dans cet exemple, différents ratios de sorbitane contre le réactif aldéhyde ont été testés. Les mêmes conditions de réaction que dans l'exemple 2 ont été utilisées, mais le rapport sorbitane: aldéhyde a été varié entre 1:1 et 3:1. Les résultats sont présentés dans le tableau 1, ci-dessous.

**Tableau 1: Effet du rapport sorbitane: aldéhyde sur le degré de conversion et le rendement isolé**

| **Rapport X/Y** | **Conversion** | **Rendement isolé (% en poids** |
|---|---|---|
| 1:1 | 96% | 62% |
| 2:1 | 81% | 83% |
| 3:1 | 95% | 89% |

Les résultats ci-dessus montrent que l'excès de sucre est avantageux en ce qu'il permet d'éviter la formation de sous-produits tels que les diacétals de sucre. Le sucre n'ayant pas réagi peut être récupéré en fin de réaction.

### Exemple 4:

Avec un ratio sorbitane:aldéhyde de 3:1 de différents réactifs aldéhydes ont été utilisés pour fournir des produits de réaction acétals de sorbitane. Les mêmes conditions de réaction et les mêmes étapes de purification comme dans l'exemple 2 ont été utilisées.

Les résultats sont présentés dans le tableau 2.

**Tableau 2:**

| **Aldéhyde** | **Conversion** | **Rendement isolé** |
|---|---|---|
| Hexanal | 100% | 98% |
| Octanal | 89% | 95% |
| Decanal | 69% | 85% |
| Dodecanal | 61% | 80% |

### Exemple 5:

Outre l'utilisation du DMF comme solvant, aussi d'autres solvants ont été utilisés pour préparer les compositions des acétals de sorbitane. Ici aussi, les mêmes réactifs ont été utilisés et la même procédure a été suivie comme dans l'exemple 2, sauf que les températures de réaction étaient aux environs de 80 ° C. Les résultats sont présentés dans le tableau 3.

**Tableau 3:**

| **Solvant** | **Conversion** | **Rendement isolé** |
|---|---|---|
| Acétonitrile | 100% | 75% |
| i-PrOH | 97% | 66% |
| DMF | 92% | 92% |

### Exemple 6:

### Acetalisation de sorbitane sans solvant:

Dans un tube scellé, le 1,4 sorbitane (X) (0,5 g, 3 mmol) a été chauffé à 95 °C. Le valeraldehyde (Y) (107 µL, 1 mmol) a été ajouté goutte à goutte, sous argon, puis de l'acide camphosulfonique (10 mg, 10% p / p) avant de refermer le tube. Le mélange est chauffé à 95 °C sous agitation magnétique. Après 15 heures, le mélange réactionnel foncé a été refroidi et dilué dans l'acétate d'éthyle (2 mL) et le solvant est ensuite évaporé sous pression réduite. Un degré de conversion de 80% a été obtenu. Le résidu a été à nouveau dilué dans l'acétate d'éthyle et l'excédent de 1,4 sorbitane a été filtré et lavé avec de l'acétate d'éthyle. Le filtrat a été concentré sous pression réduite. Le résidu est purifié par chromatographie flash (EtOAc : cyclohexane 80:20 à 100:0) pour donner l'acétal de sorbitane (0,13 g, 54% de rendement isolé) sous forme d'une huile incolore. L'HPLC a révélé un mélange de 4 isomères.

### Exemple 7:

### Trans-acetalisation du sorbitane dans l'éthanol:

Dans un ballon à fond rond du 1,4-sorbitane (0,5 g, 3 mmol) a été dissout dans de l'éthanol (7,5 mL) et le 1,1-diethoxypentane (1,15 mL, 6 mmol) a été ajouté sous un flux d'argon, puis de l'acide camphosulfonique (50 mg; 10% p/p). Le mélange est chauffé à 80 °C et sous agitation magnétique. Après 3 heures, le mélange a été neutralisé et concentré sous pression réduite. Le résidu a été purifié par chromatographie flash (acétate d'éthyle / cyclohexane 80:20 à 100:0) pour donner l'acétal de sorbitane (0,43 g, 66% de rendement isolé) sous forme d'une huile incolore. L' HPLC a révélé un mélange de 4 isomères.

### Exemple 8:

### Trans-acetalisation du sorbitane sans solvant:

Dans un ballon à fond rond, du 1,4-sorbitane (0,5 g, 3 mmol) et du 1,1-diethoxypentane (1,1-DEP) (1,15 mL, 6 mmol) (rapport molaire 1:2) ont été ajoutés sous flux d'argon, puis de l'acide camphosulfonique (50 mg; 10w / w%). Le mélange est chauffé à 80 °C sous agitation magnétique. Après 3 heures, le mélange a été directement purifié par chromatographie flash (acétate d'éthyle / cyclohexane 80:20 à 100:0) pour donner l'acétal de sorbitane (0,517 g, 73% de rendement isolé) sous forme d'une huile incolore. L'HPLC a révélé un mélange de 4 isomères. (Fig. 2)

### Exemple 9:

Les réactions de trans-acetalisation sans solvant ont été réalisées en utilisant différents rapports molaires, différents réactifs (1,1-diméthoxypentane), différentes températures de réaction et différents temps de réaction, le catalyseur étant le même. La purification des mélanges réactionnels a été réalisée par chromatographie flash, comme dans l'exemple 8.

Les résultats sont donnés dans le tableau 4.

**Tableau 4:**

| **Réactif** | **Sorbitane/réactif ratio** | **Temps (h)** | **Température** | **Conversion** | **Rendement isolé** |
|---|---|---|---|---|---|
| 1,1-DMP | 1:1 | 15 | 70°C | 99% | 66% |
| 1,1-DEP | 1:1 | 15 | 70°C | 81% | 66% |
| 1,1-DEP | 1:1 | 15 | 80°C | - | 49% |
| 1,1-DEP | 1:2 | 3 | 80°C | 80% | 73% |

Les réactions de trans-acétalisation à partir de 1,1-DMP ou de 1,1-DEP sont particulièrement pertinentes dans le cadre de réaction sans solvant où le sorbitane et le 1,1-DEP sont dans des proportions stœchiométriques..

### Exemple 10:

### Hydrogénolyse d'acétals de sorbitane:

Du pentylidene-(1,4)-sorbitane (mélange 51:49 de régioisomères, 0,98 g, 4,22 mmol) a été dilué dans du CPME sec (30 mL) et est mis dans une autoclave en acier inoxydable, avec un catalyseur 5% Pd/C (0,45 g). Le réacteur est bien fermé, purgé trois fois avec de l'hydrogène, avant que de l'hydrogène soit introduit sous pression (30 bar). Le système est chauffé à 120°C et agité pendant 15 heures. Après avoir été refroidi jusqu'à la température ambiante, l'hydrogène sous pression est relâché, le mélange de réaction est dissous dans de l'éthanol absolu (100 mL) et filtré (Filtre Millipore Durapore 0,01 micron). Le filtrat est évaporé sous pression réduite et le résidu est purifié par chromatographie flash (EtOAc/cyclohexane 90:10 à 100:0, puis EtOH/EtOAc 10:90). Ainsi un mélange d'éthers de pentyl (1,4)-sorbitane (0,686 g, 69%) a été obtenu sous forme d'une huile incolore. L'analyse par HPLC (Colonne C18, éluant eau/CH₃CN 80/20 + 0,1% v/v H₃PO₄) a montré un mélange 27:33:40 de régioisomères de pentyl(1,4)-sorbitane en position 5, 3 et 6. Les temps de rétention Rₜ sont 7,20 min (27%), 9,25 min (33%) et 10.79 min (40%) (les pics ayant été assignés respectivement aux régioisomères en position 5, 3 et 6) (Fig.3) Données spectroscopiques : ¹H NMR (400 MHz, *d*₆-DMSO) δ_{H} 0.85 (3H, t, *J* = 7), 1.20-1.37 (4H, m), 1,38-1,58 (2H, m), 3,,20-3,98 (10H, m, sorbitan protons + OCH₂ ethers), 4,02-5,15 (3H, 7m, OH protons); ¹³C NMR (100 MHz, *d*₆-DMSO) δ_{C} pour isomère majeur: 13,99 (CH₃), 22,01 (CH₂), 27,88 (CH₂), 28,99 (CH₂), 67,50 (CH), 70,59 (CH₂), 73,36 (CH₂), 73,49 (CH₂), 75,66 (CH), 76,37 (CH), 80,34 (CH). δ_{C} pour isomers mineurs: 14,02 (2 CH₃), 22,03 (2 CH₂), 27,86 and 27,91 (2 CH₂), 29,21 and 29,55 (2 CH₂), 62,02 (CH₂), 64,20 (CH₂), 68,71 (CH), 69,51 (CH₂), 69,79 (CH₂), 73,15 (CH₂), 73,23 (CH), 73,60 (CH₂), 75,53 (CH), 76,45 (CH), 77,37 (CH), 79,28 (CH), 80,10 (CH), 83,95 (CH),. HRMS (ESI⁺) calculé pour C₁₁H₂₂NaO₅: 257,1363 [M+Na]⁺; trouvé: 257,1359 (-1,4 ppm).

### Exemple 11 :

### Synthèse « one-pot » d'éthers de sorbitane à partir du 1,4-sorbitane :

Dans un ballon à fond rond de 100 mL, du 1,4-sorbitane (10 g, 62 mmol) est dissout dans du CPME sec (30 mL) en présence de Na₂SO₄ (6,5 g, 50 mmol), sous une atmosphère d'argon. Du valéraldehyde (3,3 mL, 31 mmol) est ajouté, goutte à goutte, suivi d'Amberlyst 15 (530 mg, 20 w/w% en valéraldehyde). Le mélange est chauffé jusqu'à 80°C sous agitation magnétique. Après 3 heures, le mélange chaud est filtré, lavé avec du CPME (2 x 25 mL) et le filtrat est concentré sous pression réduite. Sans purification additionnelle, le mélange est dilué dans du CPME (300 mL), séché sur du MgSO₄ et filtré. Le filtrat est introduit dans une autoclave en inox de 500 mL, et du 5%-Pd/C (3,3 mg) est ajouté. Le réacteur est bien fermé, purgé trois fois avec de l'hydrogène, avant que de l'hydrogène soit introduit sous pression (30 bar). Le système est chauffé à 120°C et agité pendant 15 heures. Après avoir été refroidi jusqu'à la température ambiante, l'hydrogène sous pression est relâché, le mélange réactionnel est dissout dans de l'éthanol absolu (250 mL) et filtré (Filtre Millipore Durapore 0,01 micron). Le filtrat est évaporé sous pression réduite et le résidu (5,8 g) est purifié par chromatographie flash (EtOAc/cyclohexane 90:10 à 100:0, puis EtOH/EtOAc 10:90). Ainsi un mélange d'éthers de pentyl(1,4)sorbitane (3,97 g, 56%) a été obtenu sous forme d'une huile incolore (pureté >98% par RMN ¹H).

### Exemple 12 :

L'octyl-1,4-sorbitan est préparé selon la procédure décrite dans l'exemple 10 à partir de octylidene-1,4-sorbitan (mélange 39:61 de régioisomères) (5,61 g, 20,4 mmol). Le résidu est purifié par chromatographie flash (EtOAc/cyclohexane 80:20 à 100:0 ensuite EtOH/EtOAc 10:90) pour obtenir un mélange d'isomères d'octyl-1,4-sorbitan comme un produit solide blanc. L'analyse par HPLC (Colonne C18, éluant eau/CH₃CN 80/20 + 0,1% v/v H₃PO₄) a montré un mélange 33 :22:45 de régioisomères d'octyl(1,4)-sorbitane en position 5, 3 et 6 (les pics ayant été assignés respectivement aux régioisomères en position 5, 3 et 6).

Données spectroscopiques : ¹H NMR (300 MHz, *d*₆-DMSO) δ_{H} 0,86 (3H, t, *J* = 7), 1,08-1,39 (10H, m), 1,39-1,58 (2H, m), 3,28-3,95 (10H, m, sorbitan protons + OCH₂ ethers), 4,02-5,10 (3H, 7m, OH protons); ¹³C NMR (75 MHz, *d*₆-DMSO): δ_{C} pour isomère majeur: 13,98 (CH₃), 22,12 (CH₂), 25,69 (CH₂), 28,73 (CH₂), 28,92 (CH₂), 29,31 (CH₂), 31,29 (CH₂), 67,48 (CH), 70,60 (CH₂), 73,35 (CH₂), 73,48 (CH₂), 75,64 (CH), 76,36 (CH), 80,33 (CH) δ_{C} pour isomères mineurs: 13,98 (2 CH₃), 22,12 (2 CH₂), 25,69 (2 CH₂), 28,88 (2 CH₂), 28,92 (2 CH₂), 28,98 (CH₂), 29,52 (CH₂), 29,88 (CH₂), 31,32 (CH₂), 62,00 (CH₂), 64,17 (CH₂), 68,69 (CH), 69,51 (CH₂), 69,82 (CH₂), 73,14 (CH₂), 73,22 (CH), 73,59 (CH₂), 75,53 (CH), 76,44 (CH), 77,37 (CH), 79,27 (CH), 80,07 (CH), 83,94 (CH) HRMS (ESI⁺) calculé pour C₁₄H₂₈NaO₅: 299.1829 [M+Na]⁺; trouvé: 299.1832 (-1.2 ppm)

### Exemple 13:

Le decyl-1,4-sorbitan est préparé selon la procédure décrite dans l'exemple 10 à partir de decylidene-1,4-sorbitan (mélange 36:64 de régioisomères) (6,12 g, 20.2 mmol). Le résidu est purifié par chromatographie flash (EtOAc/cyclohexane 70:30 à 100:0 ensuite EtOH/EtOAc 10:90) pour obtenir un mélange d'isomères de decyl-1,4-sorbitan comme un produit solide blanc. L'analyse par HPLC (Colonne C18, éluant eau/CH₃CN 50/50 + 0,1% v/v H₃PO₄) a montré un mélange 32 :16 :52 de régioisomères de decyl-(1,4)-sorbitane en position 5, 3 et 6 (les pics ayant été assignés respectivement aux régioisomères en position 5, 3 et 6).

Données spectroscopiques : ¹H NMR (300 MHz, *d*₆-DMSO) δ_{H} 0,86 (3H, t, *J* = 7), 1,09-1,38 (14H, m), 1,38-1,58 (2H, m), 3,25-4,01 (10H, m, sorbitan protons + OCH₂ ethers), 4,02-5,08 (3H, 7m, OH protons); ¹³C NMR (75 MHz, *d*₆-DMSO) δ_{C} pour isomère majeur: 13,98 (CH₃), 22,16 (CH₂), 25,76 (CH₂), 28,79 (CH₂), 29,04 (CH₂), 29,07 (CH₂), 29,14 (CH₂), 29,17 (CH₂), 29,35 (CH₂), 67,53 (CH), 70,63 (CH₂), 73,38 (CH₂), 73,50 (CH₂), 75,69 (CH), 76,40 (CH), 80,35 (CH). δ_{C} pour isomères mineurs: 13,98 (2 CH₃), 22,16 (2 CH₂), 28,98 (2 CH₂), 29,01 (2 CH₂), 29,14 (2 CH₂), 29,17 (2 CH₂), 29,35 (2 CH₂), 29,57 (2 CH₂), 29,92 (2 CH₂), 62,01 (CH₂), 64,18 (CH₂), 68,72 (CH), 69,56 (CH₂), 69,84 (CH₂), 73,16 (CH₂), 73,27 (CH), 73,60 (CH₂), 75,56 (CH), 76,48 (CH), 77,41 (CH), 79,30 (CH), 80,08 (CH), 83,96 (CH) HRMS (ESI⁺) calculé pour C₁₆H₃₂NaO₅: 327,2142 [M+Na]⁺; trouvé: 327,2135 (+2.1 ppm).

## Revendications

1. Composition d'isomères d'éthers monoalkyliques de monoanhydro-hexitol présentant un radical éther alkyle (OR) en position C-3, C-5 ou C-6 du monoanhydro-hexitol, dans lequel le groupe alkyle (R) est un groupe hydrocarboné linéaire ou ramifié comprenant entre 4 et 18 atomes de carbone, préférentiellement de 8 et 12 atomes de carbone.

2. Composition selon la revendication **1, caractérisée en ce que** le monoanhydro-hexitol est choisi parmi le monoanhydro sorbitol, le monoanhydro mannitol, le monoanhydro iditol, le monoanhydro galactitol et leur mélange, préférentiellement, le monoanhydro sorbitol ou le monoanhydro mannitol.

3. Composition selon l'une ou l'autre des revendications **1** et **2, caractérisée en ce qu'**elle comprend au moins 1% (w/w) de l'un quelconque des isomères d'éthers monoalkyliques de monoanhydro-hexitol.

4. Composition selon l'une quelconque des revendications **1** à **3, caractérisée en ce qu'**elle comprend au moins 90% (w/w), de préférence 95% d'isomères d'éthers monoalkyliques de monoanhydro-hexitol.

5. Composition selon l'une quelconque des revendications **1** à **4, caractérisée en ce que** le rapport [(3-alkyle monoanhydro-hexitol + 5-alkyle monoanhydro-hexitol) / 6-alkyle monoanhydro-hexitol] est compris entre 0,02 et 2.

6. Procédé d'obtention d'une composition d'isomères d'éthers monoalkyliques de monoanhydro-hexitol présentant un radical éther alkyle (OR) en position C-3, C-5 ou C-6 du monoanhydro-hexitol, dans lequel le groupe alkyle (R) comprend entre 4 à 18 atomes de carbone, comprenant les étapes suivantes :
a) la déshydratation d'un hexitol pour obtenir un substrat de monoanhydro-hexitol;
b) la production d'un acétal alkyle d'hexitan par acétalisation ou trans-acétalisation du substrat de monoanhydro-hexitol obtenu, avec
i. un réactif aldéhyde aliphatique comprenant de 4 à 18 atomes de carbone, par acétalisation, préférentiellement dans un rapport substrat / réactif entre 5:1 et 1:1, ou
ii. un dérivé d'un réactif aldéhyde aliphatique comprenant de 4 à 18 atomes de carbone, par trans-acétalisation, préférentiellement, dans un rapport substrat / réactif compris entre 1:1 et 1:3,
c) hydrogénolyse catalytique de l'acétal alkyle d'hexitan sans catalyseur acide, et
d) récupération d'une composition d'isomères d'éthers monoalkyliques de monoanhydro-hexitol présentant un radical éther alkyle (OR) en position C-3, C-5 ou C-6 du monoanhydro-hexitol, dans laquelle le groupe alkyle (R) comprend 4 à 18 atomes de carbone.

7. Procédé selon la revendication **6, caractérisé en ce que** l'étape b) d'acétalisation ou de trans-acétalisation est réalisée en présence d'un catalyseur acide, préférentiellement dans un environnement qui est exempt de solvant ou qui se compose d'un solvant.

8. Procédé selon l'une ou l'autre des revendications **6** et **7, caractérisé en ce que** l'hydrogénolyse est réalisée dans un solvant ou sans solvant, préférentiellement à une température se situant entre 80 et 140 °C et /ou une pression entre 15 et 40 bar, en présence d'un catalyseur, de préférence un catalyseur à base de métaux précieux.

9. Procédé selon l'une ou l'autre des revendications **7** et **8, caractérisé en ce que** le solvant est un solvant polaire, préférentiellement non-aqueux ou aprotique tel que le cyclopentyl méthyl éther (CPME).

10. Procédé selon l'une quelconque des revendications **6** à **9, caractérisé en ce qu'**il comprend au moins une étape de filtration et/ou de purification après l'une quelconque des étapes a), b) et/ou d).

11. Procédé selon la revendication **10, caractérisé en ce que** l'étape de purification s'effectue par chromatographie ou cristallisation.

12. Procédé selon les revendications **6** à **11, caractérisé en ce que** l'hexitol utilisé est choisi parmi le sorbitol et le mannitol.

13. Utilisation non-therapeutique d'une composition selon l'une quelconque des revendications **1** à **5** comme agent tensioactif non ionique, émulsifiant, lubrifiant, agent antimicrobien ou agent de dispersion.

14. Utilisation d'une composition selon l'une quelconque des revendications **1** à **5** dans la préparation d' un produit alimentaire ou non alimentaire tel qu'un produit pharmaceutique ou cosmétique.

## Patentansprüche

1. Zusammensetzung von Isomeren von Monoalkylethern von Monoanhydrohexitol, aufweisend ein Alkyletherradikal (OR) an Position C-3, C-5 oder C-6 des Monoanhydrohexitols, wobei die Alkylgruppe (R) eine lineare oder verzweigte Kohlenwasserstoffgruppe ist, die zwischen 4 bis 18 Kohlenstoffatome, vorzugsweise 8 bis 12 Kohlenstoffatome umfasst.

2. Zusammensetzung nach Anspruch **1, dadurch gekennzeichnet, dass** das Monoanhydrohexitol aus dem Monoanhydrosorbitol, dem Monoanhydromannitol, dem Monoanhydroiditol und dem Monoanhydrogalactitol und ihrem Gemisch, vorzugsweise dem Monoanhydrosorbitol oder dem Monoanhydromannitol ausgewählt ist.

3. Zusammensetzung nach einem der Ansprüche **1** und **2, dadurch gekennzeichnet, dass** sie mindestens 1 % (w/w) von einem der Isomere von Monoalkylethern von Monoanhydrohexitol umfasst.

4. Zusammensetzung nach einem der Ansprüche **1** bis **3, dadurch gekennzeichnet, dass** sie mindestens 90 % (w/w), vorzugsweise 95 % von Isomeren von Monoalkylethern von Monoanhydrohexitol umfasst.

5. Zusammensetzung nach einem der Ansprüche **1** bis **4, dadurch gekennzeichnet, dass** das Verhältnis [(3-Alkyl Monoanhydrohexitol + 5-Alkyle Monoanhydrohexitol) / 6-Alkyl Monoanhydrohexitol] zwischen 0,02 und 2 liegt.

6. Verfahren zur Herstellung einer Zusammensetzung von Isomeren von Monoalkylethern von Monoanhydrohexitol, aufweisend ein Alkyletherradikal (OR) an Position C-3, C- 5 oder C-6 des Monoanhydrohexitols, wobei die Alkylgruppe (R) zwischen 4 bis 18 Kohlenstoffatome umfasst, umfassend die folgenden Schritte:
a) Dehydratisierung eines Hexitols, um ein Monoanhydrohexitolsubstrat zu erhalten;
b) Produktion eines Hexitanacetalalkyls durch Acetalisierung oder Trans-Acetalisierung des erhaltenen Monoanhydrohexitolsubstrats mit
i. einem aliphatischen Aldehyd-Reagenz, umfassend 4 bis 18 Kohlenstoffatome, durch Acetalisierung, vorzugsweise in einem Verhältnis Substrat/Reagenz zwischen 5:1 und 1:1, oder
ii. einem Derivat eines aliphatischen Aldehyd-Reagenz, umfassend 4 bis 18 Kohlenstoffatome, durch Trans-Acetalisierung vorzugsweise in einem Verhältnis Substrat/Reagenz zwischen 1:1 und 1:3;
c) katalytische Hydrogenolyse des Hexitanacetalalkyls ohne sauren Katalysator, und
d) Rückgewinnung einer Zusammensetzung von Isomeren von Monoalkylethern von Monoanhydrohexitol, aufweisend ein Alkyletherradikal (OR) an Position C-3, C-5 oder C-6 des Monoanhydrohexitols, wobei die Alkylgruppe (R) zwischen 4 bis 18 Kohlenstoffatome umfasst.

7. Verfahren nach Anspruch **6, dadurch gekennzeichnet, dass** der Schritt b) der Acetalisierung oder Trans-Acetalisierung in Anwesenheit eines sauren Katalysators vorzugsweise in einer Umgebung durchgeführt wird, die lösungsmittelfrei ist oder die sich aus einem Lösungsmittel zusammensetzt.

8. Verfahren nach dem einen oder dem anderen der Ansprüche **6** und **7, dadurch gekennzeichnet, dass** die Hydrogenolyse in einem Lösungsmittel oder ohne Lösungsmittel durchgeführt wird, vorzugsweise bei einer Temperatur, die zwischen 80 und 140 °C liegt und/oder bei einem Druck zwischen 15 und 40 bar, in Anwesenheit eines Katalysators, vorzugsweise eines Katalysators auf der Basis von Edelmetallen.

9. Verfahren nach dem einen oder dem anderen der Ansprüche **7** und **8, dadurch gekennzeichnet, dass** das Lösungsmittel ein polares Lösungsmittel, vorzugsweise nicht wässrig oder aprotisch wie der Cyclopentylmethylether (CPME) ist.

10. Verfahren nach einem der Ansprüche **6** bis **9, dadurch gekennzeichnet, dass** es mindestens einen Filtrations- und/oder Reinigungsschritt nach einem der Schritte a), b) und/oder d) umfasst.

11. Verfahren nach Anspruch **10, dadurch gekennzeichnet, dass** der Reinigungsschritt durch Chromatographie oder Kristallisierung durchgeführt wird.

12. Verfahren nach den Ansprüchen **6** bis **11, dadurch gekennzeichnet, dass** das verwendete Hexitol aus dem Sorbitol und dem Mannitol ausgewählt ist.

13. Nicht-therapeutische Verwendung einer Zusammensetzung nach einem der Ansprüche **1** bis **5** als nichtionisches Tensid, Emulgator, Schmiermittel, antimikrobielles Mittel oder Dispersionsmittel.

14. Verwendung einer Zusammensetzung nach einem der Ansprüche **1** bis **5** für die Herstellung eines Lebensmittel- oder Nicht-Lebensmittel-Produkts wie ein pharmazeutisches oder kosmetisches Produkt.

## Claims

1. A composition of monoanhydro-hexitol monoalkyl ether isomers bearing an alkyl ether radical (OR) in position C-3, C-5 or C-6 of the monoanhydro-hexitol, in which the alkyl group (R) is a linear or branched hydrocarbon-based group comprising between 4 to 18 carbon atoms; preferably from 8 to 12 carbon atoms.

2. The composition according to claim **1, characterized in that** the monoanhydro-hexitol is selected from monoanhydro sorbitol, monoanhydro mannitol, monoanhydro iditol, monoanhydro galactitol and the mixture thereof, preferably, monoanhydro sorbitol or monoanhydro mannitol.

3. The composition according to either one of claims **1** or **2,** caracterized in that it comprises at least 1% (w/w) of any one of the monoanhydro-hexitol monoalkyl ether isomers.

4. The composition according to any one of claims **1** to **3, characterized in that** it comprises at least 90% (w/w), preferably at least 95% (w/w) of the monoanhydro-hexitol monoalkyl ether isomers.

5. The composition according to any one of claims **1** to **4, characterized in that** the ratio [(3-alkyl monoanhydro-hexitol + 5-alkyl monoanhydro-hexitol)/6-alkyl monoanhydro-hexitol] is between 0.02 and 2.

6. A process for obtaining a composition of monoanhydro-hexitol monoalkyl ether isomers bearing an alkyl ether radical (OR) in position C-3, C-5 or C-6 of the monoanhydro-hexitol, in which the alkyl group (R) is a linear or branched hydrocarbon-based group comprising between 4 to 18 carbon atoms, comprising the following steps:
a) the dehydration of a hexitol to obtain a monoanhydro-hexitol substrate;
b) the production of a hexitan alkyl acetal by acetalization or trans-acetalization of the monoanhydro-hexitol obtained substrate, with
i. an aliphatic aldehyde reagent comprising from 4 to 18 carbon atoms, by acetalization, preferably in a substrate/reagent ratio of between 5:1 and 1:1, or
ii. a derivative of an aliphatic aldehyde reagent comprising from 4 to 18 carbon atoms, by trans-acetalization, preferably in a substrate/reagent ratio of between 1:1 and 1:3,
c) the catalytic hydrogenolysis of the hexitan alkyl acetal without acid catalyst, and
d) the recovery of a composition of monoanhydro-hexitol monoalkyl ether regio-isomers, bearing an alkyl ether radical (OR) in position C-3, C-5 or C-6 of the monoanhydro-hexitol, in which the alkyl group (R) is a linear or branched hydrocarbon-based group comprising between 4 to 18 carbon atoms.

7. The process according to claim **6, characterized in that** the acetalization or trans-acetalization step b) is performed in the presence of an acid catalyst, preferably in an solvent-free environment or in the presence of a solvent.

8. The process according to either one of claims **6** and **7, characterized in that** the hydrogenolysis is performed in a solvent or without solvent, preferably at a temperature of between 80 and 140°C and/or a pressure of between 15 and 40 bar, in the presence of a catalyst, preferably a catalyst based on precious metals.

9. The process according to either one of claims **7** and **8, characterized in that** the solvent is a polar solvent, preferably non-aqueous or aprotic polar solvent such as cyclopentyl methyl ether (CPME).

10. The process according to any one of claims **6** to **9, characterized in that** it comprises at least one filtration and/or purification step after any one of steps a), b) and/or d).

11. The process according to claim **10, characterized in that** the purification step is performed by chromatography or by crystallization.

12. The process according to any one of claims **6** to **11, characterized in that** the hexitol used is selected from sorbitol and mannitol.

13. Non-therapeutic use of a composition as claimed in any one of claims **1** to **5** as a nonionic surfactant, emulsifier, lubricant, antimicrobial agent or dispersant.

14. The use of a composition as claimed in any one of claims **1** to **5** in the preparation of a food or non-food product such as a pharmaceutical or cosmetic product.
